# EUROPÄISCHE PATENTSCHRIFT

(11) **EP 1 101 505 B1**
(45) Veröffentlichungstag und Bekanntmachung des Hinweises auf die Patenterteilung: **26.03.2003**
(21) Anmeldenummer: 00123590.2
(22) Anmeldetag: 28.10.2000
(51) Int. Cl.: A61M 5/32

(54) **Verschluss für eine vorfüllbare Einmalspritze**
Cap for a pre-fillable disposable syringe
Capuchon pour une seringue pré-rempliable, à usage unique

(30) Priorität: 19.11.1999 DE 19955652
(43) Veröffentlichungstag der Anmeldung: 23.05.2001
(73) Patentinhaber: Schott Glas, 55122 Mainz (DE); CARL-ZEISS-STIFTUNG trading as SCHOTT GLAS, 55122 Mainz (DE)
(72) Erfinder: Heinz, Jochen, Dr., 24149 Kiel (DE); Spallek, Michael, Dr., 55218 Ingelheim (DE)
(74) Vertreter: Fuchs Mehler Weiss & Fritzsche

(56) Entgegenhaltungen:
- EP-A- 0 716 860
- DE-U- 8 906 101
- DE-U- 29 708 314
- US-A- 5 135 514
- US-A- 5 147 328
- US-A- 5 522 812

## Beschreibung

Die Erfindung bezieht sich auf einen Verschluß für eine vorfüllbare Einmalspritze aus Kunststoff mit einer in deren austrittsseitigem Endes des Spritzenkörpers fest verbundenen Injektionsnadel, bestehend aus einer äußeren steifen Kunststoffkappe aus hartelastischem Material, die auf den Spritzenkörper, die Injektionsnadel umhüllend, aufsetzbar ist, und die innen, zumindest im Bereich der Nadelspitze, mit einer aus einem weichelastischen Werkstoff bestehenden dichtenden Auskleidung versehen ist.

Die Erfindung bezieht sich ferner auf einen Verschluß für eine vorfüllbare Einmalspritze aus Kunststoff, mit einem an deren austrittsseitigem Ende des Spritzenkörpers angeformten Anschlußkonus einer Kegelverbindung, bestehend aus einer auf den Anschlußkonus aufgesetzten elastomeren Verschlußkappe, die von einer äußeren, mit dem Spritzenkörper verbundenen steifen Kunststoffkappe abgedeckt ist.

Vorgefüllte Einmalspritzen mit integrierter Injektionsnadel, auch Fertigspritzen genannt, seien sie aus Glas oder aus Kunststoff gefertigt, müssen während ihrer Lagerung zum Schutz der Injektionsnadel und des Spritzeninhaltes mit einer Nadelkappe versehen sein, die erst unmittelbar vor der Applikation entfernt wird. Diese Fertigspritzen werden u.a. in einschlägigen Normen beschrieben, in denen die Injektionsnadel auch als Injektionskanüle und die Nadelkappe als Schutzkappe bezeichnet wird. Sie werden typischerweise für medizinische, d.h. pharmazeutische und diagnostische Zwecke, aber auch für kosmetische Zwecke hergestellt.

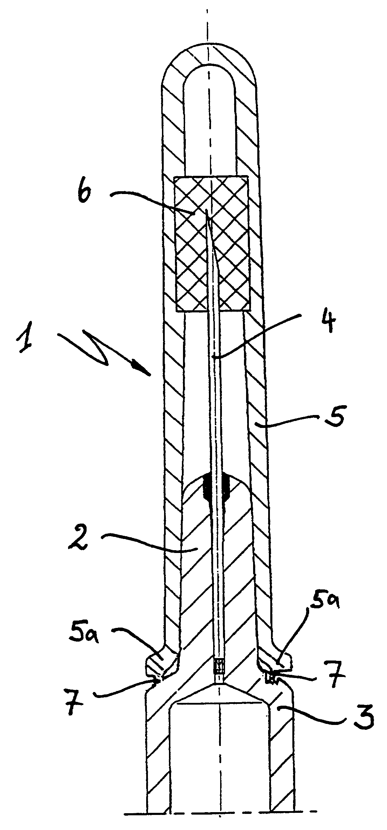

Nadelkappen sind für vorgenannte Fertigspritzen in den vielfältigsten Ausführungen bekannt bzw. auf dem Markt. Am weitesten verbreitet, sozusagen die klassische Ausführungsform, sind Nadelkappen, die aus einem Elastomer, vorzugsweise Gummi oder Naturkautschuk, hergestellt sind, wobei die Länge so groß gewählt ist, daß die Spitze der Injektionsnadel beim Aufsetzen der Kappe in die Kappenspitze einsticht und von dem Material der Kappe dichtend umschlossen bleibt. Eine derartige Nadelkappe in Verbindung mit einer im Spritzenkopf fest integrierten Injektionsnadel ist beispielsweise durch die DE-27 17 830 A1 (dortige Fig. 6) bekannt geworden. Sie besitzt auf der einen Seite die vorteilhafte Eigenschaft, daß sie einfach auf den Spritzenkopf aufstülpbar und von ihm ohne vorbereitende Schritte vollständig abziehbar ist, und dabei auch eine mikrobiologisch sichere Abdichtung gewährleistet, da sich das elastische Kappenhemd an den Spritzenkopf dichtend anschmiegt. Sie besitzt jedoch auf der anderen Seite eine Reihe von nachteiligen Eigenschaften:
- Die Nadelkappe kann infolge der Weichheit des Werkstoffes nur bedingt mechanischen Beeinträchtigungen entgegenwirken. Sie kann bei der Handhabung durch den Anwender auch durchstochen werden, was eine nicht unerhebliche Verletzungsgefahr für den Anwender beinhaltet.
- Die Nadelkappe wird beim Abziehen zusammengedrückt und legt sich eng an die Kanülenoberfläche an. Dies führt zu einem, zumindest teilweise, Abstreifen des auf die Nadel aufgebrachten Silikons, das als Gleitmittel beim Injektionsvorgang dienen soll. Fehlt nun dieses Gleitmittel, ist die Injektion mit Schmerzen beim Injektionsvorgang verbunden. Ferner können sich Silikonpartikel/-tröpfchen bilden, die so in den Körper gelangen können.
- Die automatisierte Montage der Nadelkappe ist schwierig, da eine sicher kontrollierbare Wegbegrenzung für den Aufstülpvorgang fehlt, so daß es leicht vorkommen kann, daß die Nadelkappe zu weit oder wenig weit auf den Spritzenkopf aufgesetzt wird. Beide Fälle sind äußerst nachteilig, da im ersten Fall die Nadel durch die Nadelkappe durchstechen kann, im zweiten Fall eine mikrobiologisch sichere Abdichtung nicht mehr gewährleistet ist.
- Die Injektionsnadel ist im aufgesetzten Zustand der Nadelkappe visuell durch den Anwender nicht kontrollier- bzw. beurteilbar, beispielsweise, ob die Nadel für den vorgesehenen Zweck die geeignete ist oder ob die Nadel verbogen, verunreinigt, beschädigt ist. Es muß dazu die Nadelkappe abgezogen und gegebenenfalls wieder aufgestülpt werden, was die große Gefahr der mikrobiologischen Kontamination der Nadel oder sogar des Spritzeninhalts bzw. ein Durchstechen der Nadelkappe und/oder ein Verbiegen der Nadel mit sich bringt.
- Nach einem Abziehen kann die Nadelkappe ohne weiteres wieder aufgesetzt werden, d.h. es ist keine Originalitätssicherung gewährleistet.
- Durch das Abziehen der Nadelkappe wird ein Unterdruck an der Nadelspitze erzeugt, so daß es leicht zu einem Austreten der in die Spritze gefüllten Flüssigkeit kommt. Für den Anwender ist es notwendig, diesen Flüssigkeitstropfen zu entfernen, was zum einen die tatsächliche applizierte Dosis vermindert, zum anderen wird die äußere Nadeloberfläche mit Medikament benetzt.Bei der Injektion wird dadurch Medikament undefiniert in den Einstichkanal eingebracht, was generell unerwünscht ist und bei einigen Medikamenten, z. B. Antikogulanzien (Heparin etc.) zu Blutergüssen an der Einstichstelle führen kann.

Um diese Nachteile zu vermeiden, beschreibt beispielsweise die DE 89 06 101 U eine Nadelkappe, bestehend aus einer Kunststoffkappe aus relativ hartelastischem Material, die innen, zumindest im Bereich der Kanülenspitze, mit einem weichelastischen dichtenden Werkstoff ausgekleidet ist. Hierdurch kann dieser weichelastische Werkstoff auf optimale Dichtungseigenschaften ausgewählt sein (Dichtfunktion), während der hartelastische Werkstoff geeigneter ist, mechanischen äußeren Beeinträchtigungen entgegen zu wirken (Schutzfunktion).

Von einer derartigen Nadelkappe geht die Erfindung, soweit sie sich auf eine vorfüllbare Einmalspritze mit integrierter Injektionsnadel bezieht, aus.

Neben den Kunststoff-Fertigspritzen mit integrierter Injektionsnadel sind auch solche Fertigspritzen bekannt, die am austrittsseitigen Ende des Spritzenkörpers einen daran angeformten Anschlußkonus einer Kegelverbindung, sogenannte Luer oder Luer-lock Kegelverbindungen gemäß DIN ISO 594, besitzen. Auf diesen Anschlußkonus kann typischerweise im Applikationsfall ein separater Nadelhalter aufgesetzt werden.

Der Verschluß für diesen Spritzentyp besteht typischerweise aus einer auf den Anschlußkonus aufgesetzten elastomeren Verschlußkappe, einem sogenannten Tip-Cap, die von einer äußeren steifen Kunststoffkappe aus hartelastischem Material abgedeckt ist.

Von einem derartigen Verschluß, wie er beispielsweise aus der EP 0 716 860 A2 oder der DE 195 37 163 C1 bekannt geworden ist, geht die Erfindung aus, soweit sie sich auf nadellose Spritzenkörper bezieht.

Bei beiden Spritzentypen wird das Verschlußsystem typischerweise lösbar über Hinterschnitte oder Überstände am Spritzenkörper befestigt.

Diese Befestigungsart ist fertigungstechnisch sehr aufwendig. Sie macht es notwendig, sowohl hinsichtlich des Spritzenkörpers als auch des Verschlusses mit hoher Präzision zu arbeiten, um sicherzustellen, daß z.B. nach einer Sterilisation der befüllten und verschlossenen Spritze, beispielsweise durch Autoklavieren bei 121°C/20 min gemäß den Forderungen in den Arzneibüchern, der Verschluß aufgrund thermischer Ausdehnung des Gesamtsystems nicht abfällt bzw. sich lockert, oder daß es zu einer mikrobiologischen Undichtigkeit des Verschlusses kommt.

Es kommt ein weiteres hinzu.

Vielfach besteht die Forderung, daß sichergestellt werden soll, daß die Fertigspritzen nicht wiederholt benutzbar sind. Dies führt zur Notwendigkeit eines Verschlußsystems, das sich nach erstmaligem Öffnen nicht wieder in den ursprünglichen Zustand zurückversetzen läßt.

Derartige "Originalitätsverschlüsse" sind beispielsweise für den Spritzentyp mit integrierter Nadel durch die DE 297 08 314 U1 und für den Spritzentyp mit Anschlußkonus neben der bereits erwähnten DE 195 37 163 C1 und EP 0 716 860 A2 durch die EP 0 917 882 A1 und EP 0 830 868 A2 bekannt geworden.

In allen diesen bekannten Fällen ist, wie bei den Verschlüssen ohne Originalitätssicherung, die äußere steife Kunststoffkappe auf dem Spritzenkörper durch Hinterschnitte und/oder Überstände mechanisch fixiert, mit den nachteiligen Konsequenzen, wie sie eingangs erläutert sind.

Der Erfindung liegt die Aufgabe zugrunde, einen einfachen und sicheren Verschluß für vorfüllbare Einmalspritzen aus Kunststoff mit und ohne integrierter Nadel zu schaffen, der zur mechanischen Fixierung an dem Spritzenköper keine Hinterschnitte oder Überstände erfordert, und der als Originalitätsverschluß ausgebildet ist.

Die Lösung dieser Aufgabe gelingt bei einem Verschluß gemäss den Ansprüchen 1 und 5.

Die erfindungsgemäßen Maßnahmen schaffen durch die fertigungstechnisch relativ einfach durchzuführenden Schweiß- oder Klebevorgänge eine einfache und dennoch sichere Verbindung, die den Verschluß auch bei dem bei einer Autoklavierung entstehenden erhöhten Innendruck im Behältnis sicher auf dem Behälter fixiert und so Dichtigkeit, insbesondere die mikrobiologische Dichtigkeit, gewährleistet, und die wegen der Fixierung von ebenen Flächen keine Hinterschnitte und/oder Überstände benötigt.

Ist durch Brechen der Solltrennstelle der Verschluß einmal geöffnet, kann er (mit den beim Anwender vorhandenen Mitteln) nicht wieder in den ursprünglichen Zustand zurückversetzt werden. Solltrennstellen im Rahmen von Originalitätsverschlüssen sind an sich durch eingangs zitierte Schriften bekannt geworden.

Ausgestaltende Merkmale der Erfindung sind in Unteransprüchen gekennzeichnet und werden anhand eines in der Zeichnung dargestellten Ausführungsbeispiels der Erfindung näher beschrieben.

Die einzige Figur der Zeichnung zeigt einen generell mit 1 bezeichneten, als Nadelkappe ausgebildeten Verschluß für eine vorfüllbare Einmalspritze mit einer im Spritzenkopf 2 eines Kunststoff-Spritzenkörpers 3 der Einmalspritze fest verbundenen Injektionsnadel 4. Die Nadelkappe besteht aus zwei Teilen, nämlich aus einer äußeren Kunsstoffkappe 5 aus relativ hartelastischem Material (zum Beispiel transparenten Polymeren wie Cycloolefincopolymer (COC), Polystyrol, Polyethylenterephtalat (PET), Polyethylennaphtylat (PEN), Polymethylpenten (TP), Acrylbutadienstyrol (ABS), Plycarbonat (PC) oder semitransparenten Polymeren, wie Polyethylen, Polypropylen), die innen im Bereich der Nadelspitze mit einer aus einem weichelastischen Werkstoff z.B. Naturkautschuk, Halogenbuthylelastomere, vorzugsweise einem transluzenten gummiartigen Elastomer (z.B. Silikonkautschuk, thermoplastische Elastomere, COC-Elastomere), bestehenden dichtenden Auskleidung 6 versehen ist. Die Auskleidung ist dabei formschlüssig in einer entsprechenden Ausnehmung der Kunststoffkappe 5 aufgenommen.

In einer weiteren Ausführungsform ist es möglich, mittels einem Zweikomponentenspritzgußverfahren die äußere Hülle aus einem der o.g. Kunststoffe und die Auskleidung mit einem weich-elastischen thermoplastischen Elastomer (TPE) oder Silikonkautschuk in einem Arbeitsschritt herzustellen.

Die Nadelspitze wird durch die Auskleidung 6 abgedichtet, indem die Nadel in dieses Teil beim Aufstülpen der Nadelkappe auf den Spritzenkopf 2 eingestochen wird.

Die Kunststoffkappe 5 besitzt einen umlaufenden Bund 5a. Dieser Bund 5a ist unter Ausbildung einer umlaufenden Solltrennstelle 7 über seinem gesamten Umfang mit dem Spritzenkörper 3 fest mechanisch verbunden, wobei die mechanische Verbindung auch die notwendige mikrobiologische Abdichtung sicherstellt.

Die Verbindung kann in der Weise erfolgen, daß die Kunststoffkappe 5 mit dem Kunststoff-Spritzenkörper 3 direkt verschweißt wird, z.B. durch Ultraschall-Verschweißen oder Laser-Schweißen oder Induktions-Schweißen, je nach Kunststoffmaterial bzw. Fertigungsmöglichkeiten.

Die direkte Verbindung kann auch durch Verkleben erfolgen, z.B. durch Klebstoffe, insbesondere Epoxidharze, die mit Hilfe von elektromagnetischer Strahlung ausgehärtet werden.

Diese Verbindungstechniken sind bekannt und brauchen daher hier nicht näher dargestellt zu werden.

Die vorstehende Art der Verbindung erfordert keine Hinterschnitte oder Überstände wie bei den bekannten Verschlüssen. Sie gewährleistet einen einfachen und dennoch sicheren Verschluß für Fertigspritzen ohne die eingangs geschilderten Nachteile.

Sie gewährleistet zudem einen Originalitätsverschluß, d.h. stellt ein Verschlußsystem dar, das sich nach erstmaligem Öffnen nicht wieder in den ursprünglichen Zustand zurückversetzen läßt.

In der Figur ist eine Kunststoff-Fertigspritze mit integrierter Injektionsnadel dargestellt. Die Erfindung kann jedoch auch bei Kunststoff-Fertigspritzen angewendet werden, die keine integrierte Injektionsnadel sondern einen Anschlußkonus, eine sogenannte Luer oder Luer-lock Kegelverbindung gemäß der Norm ISO 594, für einen separaten Nadelaufsatz aufweisen.

In einem derartigen Fall ist auf dem Anschlußkonus in üblicher Weise eine elastomere Verschlußkappe, ein sogenanntes "Tip-Cap", aufgesetzt, die ihrerseits mit einer harten Kunststoffkappe entsprechend der Kappe 5 in der Zeichnung abgedeckt ist. Diese harte Kunststoffkappe ist dann, wie vorstehend beschrieben, mit dem Spritzenkörper unter Ausbildung einer Solltrennstelle veschweißt oder verklebt.

Die Nadelkappe 5 in der Zeichnung besteht vorzugsweise aus einem transparenten Kunststoffmaterial, wodurch mit Vorteil eine visuelle Inspektion der Injektionsnadel bei aufgesetzter Nadelkappe möglich ist.

Der Spritzenkopf 2 und die Nadelkappe 1 sind auf vorteilhafte Weise so aufeinander abgestimmt ausgebildet, daß beim Abziehen der Kunststoffkappe 5 mit der inneren Auskleidung 6 die Öffnung in der Nadelspitze erst dann von der weichelastischen inneren Auskleidung 6 der Kunststoffkappe 5 freigegeben wird, wenn das Kappeninnere zur Herstellung eines Druckausgleiches belüftet ist, d.h. der Druck im Kappeninneren dem äußeren Luftdruck entspricht.

Dadurch ist mit großem Vorteil ein tropfenfreies Abziehen der Nadelkappe 1 möglich.

Konstruktiv wird das tropfenfreie Abziehen dadurch gewährleistet, daß die Dichtstrecke für die Nadelspitze in der Auskleidung 6 größer ist als die Dichtstrecke der Nadelkappe 1 auf dem Spritzenkopf 2.

Bei der Ausführungsform nach der Zeichnung ist die äußere Kunststoffkappe 5 allseitig geschlossen. Die Einbringung der inneren weichelastischen Auskleidung 6, die vorzugsweise aus einem gummiartigen Elastomer besteht, ist daher nur spritzenkopfseitig über die zugehörige Öffnung in der äußeren Kunststoffkappe 5 möglich.

Es sind auch Ausführungen denkbar, bei denen die äußere Kunststoffkappe 5 an der Spitze eine Ausnehmung aufweist, die durch die innere weichelastische Auskleidung 6 pfropfenartige dicht verschließbar ist.

Die Auskleidung kann dabei bündig mit der Kunststoffkappe 5 abschließen oder etwas darüber hinaus stehen.

Dadurch wird die Montage der Nadelkappe aus ihren beiden Komponenten erleichtert, beispielsweise dadurch, daß die innere Auskleidung an ihrem überstehenden Ende sicher gefaßt werden kann.

Diese Ausführungen ermöglichen eine Montage der inneren Auskleidung 6 von der Kappenspitze her, was gegebenenfalls fertigungstechnisch günstiger sein kann als die Montageart nach der Zeichnung.

## Patentansprüche

1. Verschluß (1) für eine vorfüllbare Einmalspritze aus Kunststoff mit einem Spritzenkörper (2, 3) und einer direkt in dessen austrittsseitigem Ende (2) fest verbundenen Injektionsnadel (4),
bestehend aus einer äußeren steifen Kunststoff-Nadelschutzkappe (5) aus hartelastischem Material, die direkt auf den Spritzenkörper (2, 3), die Injektionsnadel (4) umhüllend, aufsetzbar ist, und die innen, zumindest im Bereich der Nadelspitze, mit einer aus einem weichelastischen Werkstoff bestehenden dichtenden Auskleidung (6) versehen ist,
**dadurch gekennzeichnet, daß**
die Kunststoff-Nadelschutzkappe (5) mit dem Spritzenkörper (2, 3) umlaufend direkt verschweißt oder verklebt ist, unter Ausbildung einer Solltrennstelle (7) in der Schweiß- oder Klebenaht.

2. Verschluß nach Anspruch 1, **dadurch gekennzeichnet, daß** die axiale Dichtlänge der Kunststoff-Nadelschutzkappe (5) auf dem Spritzenkörper und die axiale Ausdehnung der inneren Auskleidung (6) der steifen Kunststoff-Nadelschutzkappe (5) so aufeinander abgestimmt sind, daß beim Abziehen der Nadelschutzkappe (1) das Kappeninnere zwangsbelüftet wird, bevor die Nadelspitze von der inneren Auskleidung (6) freigegeben ist.

3. Verschluß nach Anspruch 1 oder 2, **dadurch gekennzeichnet, daß** die äußere steife Kunststoff-Nadelschutzkappe (5) allseitig geschlossen ist.

4. Verschluß nach Anspruch 1 oder 2, **dadurch gekennzeichnet, daß** die äußere steife Kunststoff-Nadelschutzkappe (5) an der Spitze eine Ausnehmung (9) aufweist, die durch die innere weichelastische Auskleidung (6) dicht verschließbar ist.

5. Verschluß für eine vorfüllbare Einmalspritze aus Kunststoff, mit einem Spritzenkörper, an dessen austrittsseitigem Ende ein Anschlußkonus einer Kegelverbindung angeformt ist,
bestehend aus einer auf den Anschlußkonus aufgesetzten elastomeren Verschlußkappe, die von einer äußeren, direkt mit dem Spritzenkörper verbundenen steifen Kunststoffkappe abgedeckt ist,
**dadurch gekennzeichnet, daß**
die steife Kunststoffkappe mit dem Spritzenkörper umlaufend direkt verschweißt oder verklebt ist, unter Ausbildung einer Solltrennstelle in der Schweiß- oder Klebenaht.

## Claims

1. Cap (1) for a pre-fillable disposable syringe made of plastic with a syringe body (2, 3) and an injection needle (4) firmly connected directly in its end (2) on the outlet side,
comprising an outer rigid plastic protective needle cap (5) made of hard-elastic material, which can be fitted directly onto the syringe body (2, 3), enclosing the injection needle (4), and which is provided on the inside, at least in the region of the needle tip, with a sealing lining (6) consisting of a soft-elastic material,
**characterized in that**
the plastic protective needle cap (5) is welded or adhesively bonded directly to the syringe body (2, 3), running around the latter, thereby forming a predetermined separating location (7) in the welded or adhesively bonded joint.

2. Cap according to Claim 1, **characterized in that** the axial sealing length of the plastic protective needle cap (5) on the syringe body and the axial extent of the inner lining (6) of the rigid plastic protective needle cap (5) are made to match each other in such a way that, when the protective needle cap (5) is pulled off, the interior of the cap is forcibly ventilated before the needle tip is exposed by the inner lining (6).

3. Cap according to Claim 1 or 2, **characterized in that** the outer rigid plastic protective needle cap (5) is closed on all sides.

4. Cap according to Claim 1 or 2, **characterized in that** the outer rigid plastic protective needle cap (5) has at the tip a recess (9) which can be closed in a sealed manner by the inner soft-elastic lining (6).

5. Cap for a pre-fillable disposable syringe made of plastic, with a syringe body on the end of which, on the outlet side, a connecting cone of a tapered connection is formed,
comprising an elastomeric cap which is fitted onto the connecting cone and is covered by an outer rigid plastic cap connected directly to the syringe body,
**characterized in that**
the rigid plastic cap is welded or adhesively bonded directly to the syringe body, running around the latter, thereby forming a predetermined separating location in the welded or adhesively bonded joint.

## Revendications

1. Capuchon (1) pour une seringue pré-remplissable à usage unique en plastique, avec un corps de seringue (2, 3) et une aiguille d'injection (4) fixée directement dans l'extrémité de sortie (2) de celui-ci,
se composant d'un capuchon extérieur rigide de protection de l'aiguille en plastique (5), en matière élastique dure, qui peut être placé directement sur le corps de seringue (2, 3) en entourant l'aiguille d'injection (4), et qui est pourvu intérieurement, au moins dans la région de la pointe de l'aiguille, d'une garniture d'étanchéité (6) constituée d'une matière élastique souple,
**caractérisé en ce que**
le capuchon de protection de l'aiguille en plastique (5) est soudé ou collé en périphérie directement sur le corps de seringue (2, 3), en formant une zone de rupture préférentielle (7) dans le cordon de soudure ou de colle.

2. Capuchon suivant la revendication 1, **caractérisé en ce que** la longueur d'étanchéité axiale du capuchon de protection de l'aiguille en plastique (5) sur le corps de seringue et la dimension axiale de la garniture intérieure (6) du capuchon rigide de protection de l'aiguille en plastique (5) sont adaptées l'une à l'autre de telle façon que, lors de l'enlèvement du capuchon de protection de l'aiguille (1), l'intérieur du capuchon soit forcément aéré avant que la pointe de l'aiguille ne soit dégagée de la garniture intérieure (6).

3. Capuchon suivant la revendication 1 ou 2, **caractérisé en ce que** le capuchon extérieur rigide de protection de l'aiguille en plastique (5) est fermé de tous les côtés.

4. Capuchon suivant la revendication 1 ou 2, **caractérisé en ce que** le capuchon extérieur rigide de protection de l'aiguille en plastique (5) présente à la pointe une cavité (9), qui peut être fermée de façon étanche par la garniture intérieure élastique souple (6).

5. Capuchon pour une seringue pré-remplissable à usage unique en plastique, avec un corps de seringue à l'extrémité de sortie duquel est façonné un cône de raccordement d'un assemblage conique,
composé d'un capuchon de fermeture élastomère posé sur le cône de raccordement, qui est couvert par un capuchon en plastique rigide extérieur assemblé directement au corps de seringue,
**caractérisé en ce que** le capuchon en plastique rigide est soudé ou collé en périphérie directement sur le corps de seringue, en formant une zone de rupture préférentielle dans le cordon de soudure ou de colle.
